(19) **Europäisches Patentamt / European Patent Office / Office européen des brevets**

(11) **EP 4 574 931 A1**

(12) **EUROPEAN PATENT APPLICATION**
published in accordance with Art. 153(4) EPC

(43) Date of publication:
**25.06.2025 Bulletin 2025/26**

(21) Application number: **23854948.9**

(22) Date of filing: **18.08.2023**

(51) International Patent Classification (IPC):
*C10G 9/00* (2006.01)    *C10G 3/00* (2006.01)

(52) Cooperative Patent Classification (CPC):
**C10G 3/00; C10G 9/00**

(86) International application number:
**PCT/JP2023/029880**

(87) International publication number:
**WO 2024/038914 (22.02.2024 Gazette 2024/08)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **19.08.2022  JP 2022131236**

(71) Applicant: **Mitsubishi Chemical Corporation Tokyo 100-8251 (JP)**

(72) Inventors:
• **KIKUCHI, Satoshi**
  **Tokyo 100-8251 (JP)**
• **SHIMIZU, Toshikatsu**
  **Tokyo 100-8251 (JP)**
• **FUJISAWA, Yuusuke**
  **Tokyo 100-8251 (JP)**

(74) Representative: **Hoffmann Eitle Patent- und Rechtsanwälte PartmbB Arabellastraße 30 81925 München (DE)**

(54) **NAPHTHA FOR LOWER OLEFIN PRODUCTION AND METHOD FOR PRODUCING SAME, METHOD FOR DETERMINING NAPHTHA FOR LOWER OLEFIN PRODUCTION, LOWER OLEFIN COMPOSITION AND METHOD FOR PRODUCING SAME, AND POLYOLEFIN POLYMER**

(57)    Naphtha for producing lower olefins, the naphtha containing ether and hydrocarbons having 7 or more carbon atoms, wherein the ether has an asymmetric structure with respect to an oxygen atom constituting an ether bond, a content of the hydrocarbons having 7 or more carbon atoms is 14.0% by mass or more based on 100% by mass of a total mass of the naphtha for producing lower olefins, and a content of the ether is 20,000 ppm by mass or less in terms of ether oxygen atoms.

[Fig. 1]

# EP 4 574 931 A1

**Description**

Technical Field

The present invention relates to naphtha for producing lower olefins used in the production of lower olefins and producing method thereof.

Furthermore, the present invention relates to a producing method for a lower olefin composition that produces a lower olefin composition from the naphtha for producing lower olefins.

Furthermore, the present invention relates to a lower olefin composition obtained from the naphtha for producing lower olefins.

Furthermore, the present invention relates to a polyolefin-based polymer obtained by polymerizing the lower olefin composition.

Furthermore, the present invention relates to a method for determining naphtha for producing lower olefins.

In the present invention, the term "lower olefin" refers to an unsaturated hydrocarbon having 2 to 4 carbon atoms and containing one or two unsaturated bonds in one molecule. Examples of the lower olefin include ethylene, propylene, butenes (1-butene, 2-butene, isobutene), and butadienes (1,2-butadiene and 1,3-butadiene).

Background Art

A method of thermal cracking (steam cracking) of naphtha derived from fossil fuels (a mixture of crude oil-derived hydrocarbons having a boiling point range of about 30 to 230°C) in the presence of steam is known as a typical method for producing lower olefins (see, for example, Patent Literature 1).

In recent years, in order to achieve the Sustainable Development Goals (SDGs), efforts have been made to use renewable organic resources (such as plants) other than fossil fuels as raw materials for industrial products. One of the method proposed for this purpose is the use of biomass-derived raw materials. However, the technology of using biomass-derived raw materials, such as biomass-derived naphtha, in the naphtha cracking process is not well known.

In the steam cracking method, once the raw naphtha is selected, specific thermal cracking conditions and a specific thermal cracking device are basically required according to the composition and properties of the raw naphtha and the product requirements. Therefore, there is a drawback of poor selectivity of the raw naphtha and the product, and lack of versatility.

In other words, biomass-derived naphtha has a different composition and properties from fossil fuel-derived naphtha. For this reason, when using an existing thermal cracking device to thermally crack biomass-derived naphtha alone, or a naphtha mixture containing biomass-derived naphtha and fossil fuel-derived naphtha by steam cracking method, there is a demand to improve the production yield of low olefins from the viewpoint of improving the energy consumption rate, and the like.

Naphtha contains oxygen-containing compounds, and when naphtha is thermally cracked to produce various lower olefins, methanol derived from the thermal decomposition products of oxygen-containing compounds may be produced.

There are various oxygen-containing compounds in naphtha, however the ratio of methanol produced from these oxygen-containing compounds is not constant, and the details have not been disclosed.

When methanol derived from the thermal decomposition products of the oxygen-containing compounds is mixed with the lower olefin product such as propylene, it causes a problem of reducing the performance of the catalyst used to polymerize the lower olefin such as propylene.

For this reason, the concentration of oxygen-containing compounds in naphtha is used as a criterion for judging the quality of naphtha.

Usually, a purchaser of naphtha checks the concentration of various oxygen-containing compounds present in the naphtha before purchasing it.

The concentration of oxygen-containing compounds in naphtha is also reflected in the price of naphtha. Naphtha having a high oxygen-containing compound content is sold at a low price, while naphtha having a low oxygen-containing compound content is sold at a high price.

Due to the above background, a purchaser of naphtha usually blends purchased naphtha having a high oxygen-containing compound content with naphtha having a low oxygen-containing compound content to reduce the oxygen-containing compound content in the naphtha before using it to produce lower olefins.

Citation List

Patent Literature

Patent Literature 1: JP 2009-40913 A

Summary of Invention

Technical Problem

As described above, details about the relationship between the type of oxygen-containing compounds contained in naphtha and the amount of methanol produced from the thermal cracking products of the oxygen-containing compounds has not been clarified so far.

Therefore, even if the quality of naphtha is determined based on the concentration of oxygen-containing compounds contained in the naphtha, the concentration of methanol produced when lower olefins are actually produced by thermal cracking is not necessarily proportional to the concentration of oxygen-containing compounds in the naphtha.

In other words, a method for accurately determining high-quality naphtha having a low methanol production concentration has not been known so far.

Furthermore, a method for accurately determining high-quality naphtha having a low methanol production concentration using naphtha derived from bio raw materials, which has a different composition and properties from conventional naphtha derived from fossil fuels, has not been known so far.

The object of the present invention is to solve these problems.

In other words, the object of the present invention is to provide a naphtha for producing lower olefins that can obtain a lower olefin composition having a low methanol production concentration at a high olefin yield, a method for producing the naphtha, and a method for determining the naphtha.

Another object of the present invention is to provide a method for producing a lower olefin composition using this naphtha for producing lower olefins. Furthermore, another object of the present invention is to provide a lower olefin composition having a low methanol content produced by the method for producing a lower olefin composition, and a polyolefin-based polymer produced by polymerizing the lower olefin composition.

Solution to Problem

The present inventors have conducted extersive investigation to solve the above problems, and have come to the following findings.

(1) Among oxygen-containing compounds, certain ethers that have an asymmetric structure with respect to an oxygen atom of an ether bond are prone to selective decomposition of specific bonds in the molecule in the naphtha thermal cracking process, and are particularly prone to producing methanol through decomposition.

(2) A lower olefin composition having a low methanol production concentration can be obtained with a high olefin yield by using naphtha for producing lower olefins, in which a content of oxygen atoms derived from the specific ether having an asymmetric structure, more preferably the specific ether having an asymmetric structure and an absolute value $\Delta E$ [unit: e] of the difference in the electric charges of two carbon atoms bonded to the oxygen atom of the ether bond of the specific ether having an asymmetric structure, is a predetermined value or less, and a content ratio of hydrocarbons having 7 or more carbon atoms is a predetermined value or more.

(3) In particular, this effect is more remarkable in naphtha for producing lower olefins using naphtha derived from a bio raw material as the raw naphtha.

The present inventors have completed the present invention based on these findings.

The gist of the present invention is as follows.

[1] Naphtha for producing lower olefins, the naphtha containing ether and hydrocarbons having 7 or more carbon atoms,

wherein the ether has an asymmetric structure with respect to an oxygen atom constituting an ether bond,

a content of the hydrocarbons having 7 or more carbon atoms is 14.0% by mass or more based on 100% by mass of a total mass of the naphtha for producing lower olefins, and

a content of the ether is 20,000 ppm by mass or less in terms of ether oxygen atoms.

[2] The naphtha for producing lower olefins according to [1], wherein an average molecular weight of the hydrocarbons contained in the naphtha for producing lower olefins is 80.0 g/mol or more.

[3] The naphtha for producing lower olefins according to [1] or [2], having a specific gravity of 0.6640 g/cm$^3$ or more.

[4] The naphtha for producing lower olefins according to any one of [1] to [3], wherein the content of the ether is 0.1 ppm by mass or more in terms of ether oxygen atoms.

[5] The naphtha for producing lower olefins according to any one of [1] to [4], wherein a content of sulfur-containing compounds is 180 ppm by mass or less in terms of sulfur atoms.

[6] The naphtha for producing lower olefins according to any one of [1] to [5], wherein the content of the hydrocarbons having 7 or more carbon atoms is 42.0% by mass or less based on 100% by mass of a total mass of the naphtha for producing lower olefins.

[7] The naphtha for producing lower olefins according to any one of [1] to [6], wherein an average molecular weight of the hydrocarbons contained in the naphtha for producing lower olefins is 87.0 g/mol or less.

[8] The naphtha for producing lower olefins according to any one of [1] to [7], having a specific gravity of 0.6695 g/cm$^3$ or less.

[9] The naphtha for producing lower olefins according to any one of [1] to [8], wherein a content of sulfur-containing compounds is 1 ppm by mass or more in terms of sulfur atoms.

[10] The naphtha for producing lower olefins according to any one of [1] to [9], wherein the naphtha for producing lower olefins includes naphtha derived from a bio raw material.

[11] The naphtha for producing lower olefins according to [10], wherein the naphtha for producing lower olefins is naphtha derived from a bio raw material alone, or a naphtha mixture including naphtha derived from a bio raw material and naphtha derived from a fossil fuel.

[12] The naphtha for producing lower olefins according to [10] or [11], wherein the naphtha derived from a bio raw material is naphtha derived from a non-edible biomass and/or a non-fossil fuel.

[13] The naphtha for producing lower olefins according to any one of [1] to [12], wherein the ether has an absolute value ΔE (ΔE=|E1-E2|) of the difference between a charge E1 of one carbon atom and a charge E2 of another carbon atom, calculated by the density functional theory method for two carbon atoms bonded to an oxygen atom constituting an ether bond, of 0.05 [unit: e] or more,
wherein e means the elementary charge, and e = 1.602176634 x 10$^{-19}$ [unit: C].

[14] The naphtha for producing lower olefins according to any one of [1] to [13], wherein the ether is monoether.

[15] The naphtha for producing lower olefins according to any one of [1] to [14], wherein one of two carbon atoms bonded to an oxygen atom constituting an ether bond of the ether is a carbon atom of a methyl group.

[16] The naphtha for producing lower olefins according to any one of [1] to [15], wherein the lower olefin is propylene.

[17] A method for producing a lower olefin composition, the method comprising a step of cracking the naphtha for producing lower olefins according to any one of [1] to [16].

[18] The method for producing a lower olefin composition according to [17], wherein the naphtha for producing lower olefins is cracked to produce a lower olefin composition containing lower olefins and methanol.

[19] The method for producing a lower olefin composition according to [18], wherein the lower olefin contained in the lower olefin composition includes propylene.

[20] A lower olefin composition comprising a lower olefin and/or a derivative thereof, the composition being a cracking product of the naphtha for the producing lower olefins according to any one of [1] to [16].

[21] The lower olefin composition according to [20], wherein the lower olefin contained in the lower olefin composition is an unsaturated hydrocarbon having 2 to 4 carbon atoms and containing one or two unsaturated bonds in one molecule.

[22] The lower olefin composition according to [20] or [21], further comprising methanol.

[23] A polyolefin-based polymer obtained by polymerizing the lower olefin and/or the derivative thereof contained in the lower olefin composition according to any one of [20] to [22].

[24] A method for producing naphtha for producing lower olefins, comprising blending naphtha derived from a bio raw material and naphtha derived from a fossil fuel to obtain the naphtha for producing lower olefins according to any one of [1] to [16].

[25] A method for determining naphtha for producing lower olefins,

comprising blending naphtha derived from a bio raw material and naphtha derived from a fossil fuel to obtain a blended naphtha,

determining that the blended naphtha, when it corresponds to the naphtha for producing lower olefins according to any one of [1] to [16], is acceptable as a naphtha composition to be used in producing lower olefins, and subjecting it to a thermal cracking process.

Advantageous Effects of Invention

The naphtha for producing lower olefins of the present invention produces a low amount of methanol during thermal cracking, and therefore can produce a lower olefin composition having a low methanol content with a high olefin yield.

According to the present invention, the quality of the naphtha as a raw material for lower olefins can be accurately determined from the content of oxygen atoms derived from the specific ether in the naphtha for producing lower olefins, in regard to raw naphtha, particularly in naphtha for producing lower olefins containing naphtha derived from a bio raw material as a raw material. Specifically, the specific ether has an absolute value $\Delta E$ ($\Delta E = |E1-E2|$) of the difference between a charge E1 of one carbon atom and a charge E2 of another carbon atom, calculated by the density functional theory method for two carbon atoms bonded to an oxygen atom constituting an ether bond, of 0.05 [unit: e] or more. The naphtha for producing lower olefins can be easily realized according to the present invention.

For this reason, it is possible to produce high purity lower olefins using inexpensive raw naphtha while suppressing the methanol production concentration by selecting naphtha having a low content of oxygen atoms derived from ethers having the $\Delta E$ of 0.05 [unit: e] or more from among naphthas that are inexpensive due to their high content of oxygen-containing compound. Furthermore, even if the naphtha has a high concentration of oxygen-containing compounds, as long as it has a low content of oxygen atoms derived from ethers having the $\Delta E$ of 0.05 [unit: e] or more, it can be used as the raw naphtha without blending it with naphtha having a low concentration of oxygen-containing compounds.

Brief Description of Drawings

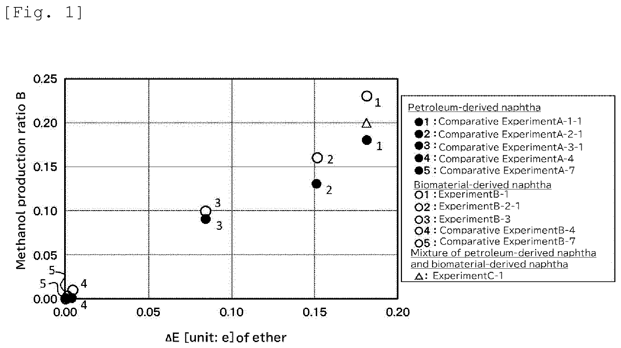

[Fig. 1] Fig. 1 is a graph showing the relationship between the $\Delta E$ [unit: e] of various ethers and the methanol production ratio B.

Description of Embodiments

The present invention will be described in detail below. The present invention is not limited to the following description as long as it does not exceed the gist of the invention, and can be carried out with any modification within the scope of the gist of the present invention.

Unless otherwise specified, in this specification, a numerical range expressed using "to" means a range including the numerical values written before and after "to" as the lower and upper limits. "A to B" means A or more and B or less.

In the present invention, the term "lower olefin" refers to an unsaturated hydrocarbon having 2 to 4 carbon atoms and containing one or two unsaturated bonds in one molecule. Examples of the lower olefin include ethylene, propylene, butenes (1-butene, 2-butene, isobutene), and butadienes (1,2-butadiene and 1,3-butadiene).

[Naphtha for producing lower olefins]

The naphtha for producing lower olefins of the present invention contains ether and hydrocarbons having 7 or more carbon atoms. The ether has an asymmetric structure with respect to an oxygen atoms constituting an ether bond (hereinafter, such ethers may be referred to as "asymmetric ether"). A content of the hydrocarbons having 7 or more carbon atoms is 14.0% by mass or more based on 100% by mass of a total mass of the naphtha for producing lower olefins, and a content of the ether is 20,000ppm by mass or less in terms of ether oxygen atoms.

Since the amount of methanol produced by a thermal cracking is small in such naphtha for producing lower olefins, a lower olefin composition having a low methanol content can be produced with a high olefin yield. The naphtha for producing lower olefins of the present invention is particularly suitable for producing propylene as a lower olefin.

Here, the term "ether oxygen atom" refers to an oxygen atom involved in an ether bond contained in the ether.

<Hydrocarbons having7 or more carbon atoms>

Examples of the hydrocarbons having 7 or more carbon atoms contained in the naphtha for producing lower olefins of the present invention include aromatic hydrocarbons such as ethylbenzene, styrene, and the like; aliphatic hydrocarbons such as normal heptane, normal octane, normal decane, and the like; and naphthenes such as methylcyclohexane, ethylcyclohexane, and the like; which are mainly contained in naphtha derived from biomass. The upper limit of the carbon number of these hydrocarbons is usually 15 or less.

The naphtha for producing lower olefins of the present invention may contain only one of these hydrocarbons having 7 or more carbon atoms, or may contain two or more of them.

The naphtha for producing lower olefins of the present invention has a content ratio of these hydrocarbons having 7 or more carbon atoms of 14.0% by mass or more based on 100% by mass of a total mass of the naphtha for producing lower olefins. When the content of the hydrocarbons having 7 or more carbon atoms is 14.0% by mass or more, the effect of specifying the upper limit of the content of the asymmetric ethers in the naphtha for producing lower olefins can be effectively obtained. Specifically, a lower olefin composition having a lower methanol content concentration can be produced with a higher olefin yield.

From this viewpoint, the lower limit of the content of the hydrocarbons having 7 or more carbon atoms in the naphtha for producing lower olefins of the present invention is 14.0% by mass or more, preferably 16.0% by mass or more, more preferably 18.0% by mass or more, and even more preferably 20.0% by mass or more.

On the other hand, when the content of high carbon number hydrocarbons in the naphtha increases too much, there is a problem that the yield of low olefins such as ethylene, propylene, and the like decreases. From this viewpoint, the upper limit of the content of the hydrocarbons having 7 or more carbon atoms in the naphtha for producing low olefins of the present invention is preferably 42.0% by mass or less, more preferably 38.0% by mass or less, and even more preferably 35.0% by mass or less.

The above upper and lower limits can be combined in any combination. That is, the content of the hydrocarbons having 7 or more carbon atoms in the naphtha for producing low olefins of the present invention is preferably 14.0% by mass or more and 42.0% by mass or less, more preferably 16.0% by mass or more and 38.0% by mass or less, even more preferably 18.0% by mass or more and 35.0% by mass or less, and particularly preferably 20.0% by mass or more and 35.0% by mass or less.

In the naphtha for producing lower olefins of the present invention, when the content of the hydrocarbons having 7 or more carbon atoms is 14.0% by mass or more and the content of the asymmetric ether is 20,000ppm by mass or less in terms of ether oxygen atoms, the effect for producing a lower olefin composition having a low methanol content concentration with a high olefin yield is significantly achieved. The reason for this is unclear, however it is presumed as follows.

That is, when naphtha contains the hydrocarbons having 7 or more carbon atoms, which are relatively large molecular weight hydrocarbons, the partial pressure of these hydrocarbons tends to be low. As a result, the side reaction for producing methanol from the asymmetric ether is suppressed. For this reason, it is presumed that the amount of methanol produced during thermal cracking can be reduced.

The content of the hydrocarbons having 7 or more carbon atoms in the naphtha for producing lower olefins can be analyzed by the method described in the Examples section below.

Although the lower limit of the average molecular weight of the hydrocarbons in the naphtha for producing lower olefins of the present invention is not particularly limited, it is preferably 80.0 g/mol or more, more preferably 81.5 g/mol or more, and even more preferably 83.0 g/mol or more from the viewpoint for producing a lower olefin composition having a low methanol content concentration with a high olefin yield. On the other hand, although the upper limit of the average molecular weight of the hydrocarbons is not particularly limited, when the content of high carbon number hydrocarbons in the naphtha increases too much, the yield of lower olefins such as ethylene, propylene, and the like tends to decrease. For this reason, the upper limit is preferably 87.0 g/mol or less, more preferably 86.5 g/mol or less, and even more preferably 86.0 g/mol or less.

The above upper and lower limits can be combined arbitrarily. That is, the average molecular weight of the hydrocarbons in the naphtha for producing lower olefins of the present invention is not particularly limited, however it is preferably 80.0 g/mol or more and 87.0 g/mol or less, more preferably 81.5 g/mol or more and 86.5 g/mol or less, and even more preferably 83.0 g/mol or more and 86.0 g/mol or less.

The average molecular weight of the hydrocarbons in the naphtha for producing lower olefins can be analyzed by the method described in the Examples section below.

<Asymmetric ether>

The naphtha for producing lower olefins of the present invention has a content of the asymmetric ether having an asymmetric structure with respect to an oxygen atoms constituting an ether bond (hereinafter sometimes referred to as "ether oxygen atoms") of 20,000 ppm by mass or less in terms of ether oxygen atoms.

When the content of the asymmetric ether in the naphtha for producing lower olefins is 20,000 ppm by mass or less in terms of ether oxygen atoms, the content of methanol in the lower olefin composition obtained by thermally cracking the naphtha for producing lower olefins can be reduced for reasons described below.

In the present invention, the content of methanol in the lower olefins obtained by thermally cracking the naphtha for producing lower olefins of the present invention can be more effectively reduced, when the asymmetric ether has an absolute value $\Delta E$ ($\Delta E=|E1-E2|$) of the difference between a charge E1 of one carbon atom and a charge E2 of another carbon atom, calculated by the density functional theory method for two carbon atoms bonded to an oxygen atom constituting an ether bond, of 0.05 [unit: e] or more; and when a content of the asymmetric ether is 20,000 ppm by mass or less in terms of ether oxygen atoms. The reasons will be described below.

In this specification, "e" means the elementary charge, and e = 1.602176634 x $10^{-19}$ [unit: C].

The charge values of the two carbon atoms are calculated by the density functional theory method (DFT) calculations on the molecular structure of the ether. For the DFT calculation conditions, def-TZVP is used as the basis set, the COSMO solvation model (COnductor-like Screening MOdel) is used as the solvent effect, and the Mulliken's charge density analysis method (Population Analysis) can be used as the analysis method.

To calculate the $\Delta E$, the quantum chemistry calculation software "TURBOMOLE" (manufactured by TURBOMOLE) and the graphical user interface for TURBOMOLE "TmoleX" (manufactured by TURBOMOLE) can be used.

Generally, ethers having a large $\Delta E$ are asymmetric ethers, which have an asymmetric structure with respect to the ether oxygen atom. The present inventors have found that asymmetric ethers tend to be easily decomposed to produce methanol under thermal cracking conditions.

Furthermore, the present inventors have found that the larger the $\Delta E$ of ether, the more charge imbalance there is between the two carbon atoms bonded to the ether oxygen atom, and therefore such ether tends to be easily decomposed to produce methanol under thermal cracking conditions.

Furthermore, the present inventors have found that by using the asymmetric ether as the ether, more preferably the asymmetric ether having the $\Delta E$ [unit: e] of 0.05 or more, and by setting the content of the asymmetric ether contained in the naphtha to 20,000 ppm by mass or less in terms of ether oxygen atoms, it is possible to reduce the methanol content concentration in the lower olefin composition obtained by thermally cracking the naphtha.

Examples of the asymmetric ether having an asymmetric structure with respect to the ether oxygen atom include 2-methoxybutane ($CH_3CH_2CH(CH_3)$-O-$CH_3$), methoxycyclopentane ($C_5H_9$-O-$CH_3$), and 1-methoxypropane ($CH_3CH_2CH_2$-O-$CH_3$). These ethers have the $\Delta E$ [unit: e] of 0.181, 0.151, and 0.084, respectively, which means that the $\Delta E$ is large and the charge imbalance is large. For this reason, a specific bond in the molecule is easily decomposed selectively. As a result, the ethers tend to produce methanol in the thermal cracking process of the naphtha containing these ethers.

On the other hand, ethers having a symmetric structure with respect to the ether oxygen atom (hereinafter referred to as "symmetric ether"), such as dimethyl ether ($CH_3$-O-$CH_3$), diethyl ether ($CH_3$-$CH_2$-O-$CH_2$-$CH_3$), diisopropyl ether (($CH_3$)$_2$CH-O-CH($CH_3$)$_2$), and dipropyl ether ($CH_3$-$CH_2$-$CH_2$-O-$CH_2$-$CH_2$-$CH_3$) have the $\Delta E$ [unit: e] of 0.004, 0.001, 0.010, and 0.000, respectively. This means that the $\Delta E$ is small and the charge imbalance is small. For this reason, it is difficult for a specific bond in the molecule to be selectively decomposed. As a result, in the thermal cracking process of the naphtha containing these symmetric ethers, the symmetric ethers are unlikely to produce methanol.

The naphtha for producing lower olefins of the present invention has an asymmetric ether content of a predetermined value or less, so that the content of produced methanol in the lower olefin composition obtained by thermal cracking it can be reduced.

The asymmetric ether is preferably a monoether having only one ether oxygen atom in the molecule, since it can effectively reduce the content of produced methanol in the obtained lower olefin composition.

The asymmetric ether having the ΔE [unit: e] of 0.05 or more has the ΔE value of 0.05 or more for at least one ether bond in the molecule. There is a high correlation between the ΔE value and the amount of produced methanol. The asymmetric ether is preferably a monoether having only one ether oxygen atom in the molecule, since it can effectively reduce the content of produced methanol in the obtained lower olefin composition.

The asymmetric ether, preferably the asymmetric ether having the ΔE of 0.05 or more, is preferably one wherein one of the two carbon atoms bonded to the ether oxygen atom is a carbon atom of a methyl group, such as 2-methoxybutane, methoxycyclopentane, and 1-methoxypropane. Such asymmetric ether can more effectively reduce the content of produced methanol in the obtained lower olefin composition.

When the ether has two or more ether bonds in one molecule, the ΔE of each ether bond is calculated as the absolute value of the difference in charge between the two carbon atoms bonded to each ether oxygen atom. The ΔE of the ether is the largest value among the two or more ΔE.

In the naphtha for producing lower olefins of the present invention, the upper limit of the content of the asymmetric ether is 20,000 ppm by mass or less, preferably 10,000 ppm by mass or less, more preferably 1,000 ppm by mass or less, even more preferably 100 ppm by mass or less, particularly preferably 50 ppm by mass or less, and most preferably 30 ppm by mass or less, in terms of ether oxygen atoms, from the viewpoint of suppressing the amount of methanol produced in the lower olefin composition obtained by thermally cracking the naphtha.

Although the lower limit of the content of the asymmetric ether contained in the naphtha for producing lower olefins of the present invention is not particularly limited, the content is usually 0.1 ppm by mass or more, preferably 0.3 ppm by mass or more, more preferably 1.0 ppm by mass or more, even more preferably 3.0 ppm by mass or more, particularly preferably 7.0 ppm by mass or more, and most preferably 12.0 ppm by mass or more, in terms of ether oxygen atoms from the viewpoint of the production cost for reducing the asymmetric ether and from the viewpoint of the quantitative accuracy by GC and GC/MS measurements, which are general analytical instruments.

The above upper and lower limits can be combined in any combination. That is, the content of the asymmetric ether contained in the naphtha for producing lower olefins of the present invention is not particularly limited, but is usually 0.1 ppm by mass or more and 20,000 ppm by mass or less, preferably 0.3 ppm by mass or more and 10,000 ppm by mass or less, more preferably 1.0 ppm by mass or more and 1,000 ppm by mass or less, even more preferably 3.0 ppm by mass or more and 100 ppm by mass or less, particularly preferably 7.0 ppm by mass or more and 50 ppm by mass or less, and most preferably 12.0 ppm by mass or more and 30 ppm by mass or less, in terms of ether oxygen atoms.

The content of the asymmetric ether in the naphtha for producing lower olefins in terms of ether oxygen atoms can be determined based on the asymmetric ether contained in the naphtha using general analytical instrument such as GC and GC/MS measurements, and the like.

<Sulfur-containing compound>

Naphtha usually contains sulfur-containing compounds such as disulfide compounds, sulfide compounds, thiol compounds, and the like. In the naphtha for producing lower olefins of the present invention, the upper limit of the content of the sulfur-containing compounds is not particularly limited, but the content is preferably 180 ppm by mass or less, more preferably 150 ppm by mass or less, even more preferably 100 ppm by mass or less, particularly preferably 30 ppm by mass or less, and most preferably 10 ppm by mass or less, in terms of sulfur atoms, since a lower olefin composition having a low methanol content can be produced with a high olefin yield.

The content of the sulfur-containing compounds is preferably as small as possible. There is no particular limit to the lower limit of the content of the sulfur-containing compounds, but from the viewpoint of production costs for reducing the sulfur-containing compounds, the content is usually preferably 1 ppm by mass or more, more preferably 3 ppm by mass or more, even more preferably 5 ppm by mass or more, and particularly preferably 7 ppm by mass or more.

The reason why a lower content of the sulfur-containing compounds allows a lower olefin composition having a lower methanol content to be produced having a higher olefin yield is unclear, however it is presumed that the sulfur-containing compounds promote side reactions.

The upper and lower limits above can be combined in any combination. That is, the content of the sulfur-containing compounds in the naphtha for producing lower olefins of the present invention is not particularly limited, but is preferably 1 ppm by mass or more and 180 ppm by mass or less, more preferably 3 ppm by mass or more and 150 ppm by mass or less, even more preferably 5 ppm by mass or more and 100 ppm by mass or less, particularly preferably 7 ppm by mass or more and 30 ppm by mass or less, and most preferably 7 ppm by mass or more and 10 ppm by mass or less, in terms of sulfur atoms.

Specific examples of the disulfide compound include dimethyl disulfide, methyl ethyl disulfide, diethyl disulfide, and the like.

Specific examples of the sulfide compound include diethyl sulfide, dipropyl sulfide, and the like.

Specific examples of the thiol compound include thiol compounds having 2 to 10 carbon atoms, such as ethanethiol, propanethiol, butanethiol, and the like.

The naphtha for producing lower olefins may contain only one type of these sulfur-containing compounds, or two or more types.

<Specific gravity>

The specific gravity of the naphtha for producing lower olefins of the present invention is not particularly limited. It is known that there is a correlation between naphtha density and naphtha composition. Since a lower olefin composition having a low methanol content concentration can be produced with a high olefin yield, the lower limit of the specific gravity is preferably 0.6640 g/cm$^3$ or more, and more preferably 0.6650 g/cm$^3$ or more.

The upper limit of the specific gravity is preferably 0.6695 g/cm$^3$ or less, and more preferably 0.6680 g/cm$^3$ or less, since the yield of lower olefins such as ethylene, propylene, and the like can be maintained well.

The above upper and lower limits can be combined in any combination. That is, the specific gravity of the naphtha for producing lower olefins of the present invention is not particularly limited, however it is preferably 0.6640 g/cm$^3$ or more and 0.6695 g/cm$^3$ or less, and more preferably 0.6650 g/cm$^3$ or more and 0.6680 g/cm$^3$ or less.

The reason why a lower olefin composition having a low methanol concentration can be produced with a higher olefin yield when the specific gravity of the naphtha for producing lower olefins is within the above mentioned numerical range is not clear, however it is presumed that the higher the naphtha density, the greater the tendency for the number of carbon atoms in the hydrocarbons contained in the naphtha to increase, resulting in efficient cracking of the naphtha into olefins.

The specific gravity of the naphtha can be measured in accordance with JIS K2249-1:2011.

<Raw naphtha>

The naphtha for producing lower olefins of the present invention preferably contains naphtha derived from a bio raw material, from the viewpoint of being able to suppress the production of methanol produced from the asymmetric ethers contained as impurities and to obtain a high yield of lower olefins.

The naphtha for producing lower olefins of the present invention is preferably a naphtha derived from a bio raw material alone, or a naphtha mixture containing naphtha derived from a bio raw material and naphtha derived from a fossil fuel, from the viewpoint of being able to suppress the production of methanol produced from the asymmetric ethers contained as impurities and to obtain a high yield of lower olefins.

In the present invention, the naphtha derived from a bio raw material is naphtha derived from a non-edible biomass and/or a non-fossil fuel.

In the present invention, the non-edible biomass refers to resources made from non-edible grasses and trees. Specific examples of such materials include cellulose, hemicellulose, lignin, and the like obtained from woody biomass such as conifers and broadleaf trees, bioethanol and biodiesel obtained from herbaceous biomass such as corn and sugarcane stalks, soybeans, rapeseed, and the like; plant-based waste oil; and the like, but are not limited to these.

In the present invention, the non-fossil fuel refers to, for example, hydrogen, or organic matter derived from animals and plants that is not derived from the fossil fuels or the non-edible biomass. Specific examples of such materials include methane, sugar ethanol, and the like obtained from firewood, charcoal, dried livestock dung, and the like, but are not limited to these.

In the present invention, naphtha derived a from fossil fuel refers to at least one type selected from naphtha derived from petroleum, naphtha derived from coal, and naphtha derived from natural gas.

<Method for producing naphtha for producing lower olefins>

The method for producing the naphtha for producing lower olefins of the present invention is not particularly limited. A specific embodiment includes a method of mixing naphtha derived from a bio raw material with naphtha derived from a fossil fuel.

More specific embodiments include a method wherein naphtha derived from a bio raw material and naphtha derived from a fossil fuel are mixed to obtain a blended naphtha, and the naphtha for producing lower olefins of the present invention, in which the mixed naphtha contains the asymmetric ether having the asymmetric structure with respect to the oxygen atom constituting the ether bonds as ether, and the content of the asymmetric ether, the content ratio of the hydrocarbons having 7 or more carbon atoms, and more preferably the specific gravity of the naphtha and the content of the sulfur-containing compounds are within the above mentioned preferred ranges, is produced as the blended naphtha.

In this case, one or both of the following methods can be used to bring the content of the asymmetric ether within the above mentioned preferred ranges.

    (1) Purchase or obtain naphtha derived from a fossil fuel or naphtha derived from a bio raw material, wherein the content of the asymmetric ether is 20,000 ppm by mass or less in terms of ether oxygen atoms.

(2) When the naphtha purchased or obtained has a high asymmetric ether content, the content is reduced by mixing it with naphtha or the like having a low asymmetric ether content.

In order to bring the content ratio of the hydrocarbons having 7 or more carbon atoms into the above mentioned preferred range, naphtha derived from a bio raw material can be appropriately mixed with naphtha derived from a fossil fuel.

In order to bring the specific gravity of naphtha for producing lower olefins into the above mentioned preferred range, naphtha derived from a bio raw material can be appropriately mixed with naphtha derived from a fossil fuel.

In order to bring the content of the sulfur-containing compounds into the above mentioned preferred range, naphtha derived from a bio raw material can be appropriately mixed with naphtha derived from a fossil fuel.

[Method for determining naphtha for producing lower olefins]

The method for determining naphtha for producing lower olefins, which is one embodiment of the present invention, is a method for determining naphtha, which includes, in producing naphtha for producing lower olefins of the present invention, blending naphtha derived from a bio raw material and naphtha derived from a fossil fuel to obtain a blended naphtha, and then, when the blended naphtha obtained corresponds to the naphtha for producing lower olefins of the present invention described above, determining that the blended naphtha is acceptable as a naphtha composition to be used in producing lower olefins, and subjecting the blended naphtha to a thermal cracking process.

More specifically, the method for determining naphtha for producing lower olefins, which is one embodiment of the present invention, is a method, when a blended naphtha contains the asymmetric ether having the asymmetric structure with respect to the oxygen atom constituting the ether bond as the ether, and the content of the asymmetric ether, the content ratio of the hydrocarbon having 7 or more carbon atoms, and more preferably the specific gravity of the blended naphtha and the content of the sulfur-containing compound are within the above mentioned preferred ranges, determining the blended naphtha to be acceptable as a naphtha composition to be used for producing lower olefins, and subjecting the blended naphtha to a thermal cracking process.

[Method for producing lower olefin composition]

The method for producing a lower olefin composition of the present invention includes a step of cracking the naphtha for producing lower olefins of the present invention.

A lower olefin composition containing lower olefins and methanol is produced by thermally cracking the naphtha for producing lower olefins of the present invention.

In this method for producing the lower olefin composition, the amount of methanol produced can be significantly reduced as described above by using the naphtha for producing lower olefins of the present invention as the naphtha for producing lower olefins to be subjected to thermal cracking.

The method for producing a lower olefin composition of the present invention can produce a lower olefin composition by the same process according to a conventional method, except that the naphtha for producing lower olefins of the present invention is used.

That is, the naphtha for producing lower olefins of the present invention (hereinafter sometimes simply referred to as "naphtha") is thermally cracked (steam cracked) at a temperature of 700 to 1000°C in the presence of steam to obtain a lower olefin composition.

Among the conditions for thermal cracking, the ratio of naphtha to steam is preferably 20 to 100 parts by mass of steam more preferably 30 to 70 parts by mass of steam, and particularly preferably 35 to 60 parts by mass of steam, based on 100 parts by mass of naphtha. When the amount of steam is less than 20 parts by mass, there is a tendency to increase carbonaceous materials deposition on the piping installed in the thermal cracking furnace for carrying out the cracking reaction. On the other hand, when the amount of steam exceeds 100 parts by mass, the amount of heat given to the steam increases, and the energy load on the thermal cracking furnace tends to increase.

The reaction temperature of the thermal cracking is usually 700 to 1000°C, and preferably 750 to 950°C. When the reaction temperature is lower than 700°C, the thermal cracking of naphtha does not proceed sufficiently, and the yield of the desired low olefins decreases. When the reaction temperature exceeds 1000°C, the thermal cracking of naphtha becomes excessive, and the generation of undesirable by-products such as methane increases, and the yield of the desired low olefins tends to decrease.

The reaction time of the thermal cracking is preferably 0.01 to 1 second, more preferably 0.04 to 0.7 seconds. When the reaction time is less than 0.01 second, the thermal cracking of naphtha does not proceed sufficiently, and the yield of the desired low olefins tends to decrease. When the reaction time exceeds 1 second, the thermal cracking of naphtha becomes excessive, and the generation of undesirable by-products such as methane increases, and the yield of the desired low olefins tends to decrease.

The reaction pressure of the thermal cracking is preferably 0.01 to 1.5 MPa (gauge pressure), more preferably 0.05 to 0.5 MPa (gauge pressure), and even more preferably 0.07 to 0.2 MPa (gauge pressure).

The reaction product that has left the reaction zone of the thermal cracking can be rapidly cooled to prevent excessive cracking. Although the cooling temperature is not particularly limited, when the cracking is carried out on an industrial scale for example, it is preferably 200 to 700°C, and more preferably 250 to 650°C. When the cracking is carried out on a small scale such as a pilot, laboratory, or the like, it is preferably 0 to 100°C, and more preferably 3 to 40°C.

The reaction product containing the lower olefin thus obtained can be subjected to treatments such as purification, fractional distillation, and the like, according to conventional methods. As a result, lower olefins such as ethylene, propylene, butene, butadiene, and the like, aromatic hydrocarbons, and other hydrocarbons are obtained, respectively.

Saturated hydrocarbons such as ethane, propane, and the like can be recovered and subjected to thermal cracking again.

Butene and butadiene among the lower olefins are usually obtained as a mixture with butane. Therefore, butadiene is isolated by solvent extraction in an other process. It is preferable that the mixture of butene and butane remaining after extraction is utilized or fractionated by polymerization, rectification or the like in an other process.

In the method for producing a lower olefin composition of the present invention, it is preferable that the $\Delta E$ [unit: e] and the methanol production ratio B in the obtained lower olefin satisfy the following formulas (1) and (2). This makes it possible to obtain a lower olefin composition having a lower methanol content by the thermal cracking of naphtha for producing lower olefins of the present invention.

$$0.05 \leqq \Delta E \qquad \text{Formula (1)}$$

$$B \leqq 1.25 \times \Delta E + 0.10 \qquad \text{Formula (2)}$$

As mentioned above, methanol has an adverse effect on the polymerization catalyst when polymerizing lower olefins. The lower olefins obtained by cracking naphtha under the conditions satisfying the above formulas (1) and (2) have a reduced methanol content. Therefore, it is effective in producing lower olefins such as propylene.

It is more preferable that the above formula (2) satisfies the condition $B \leqq 1.25 \times \Delta E + 0.05$.

The above methanol production ratio B is an index showing the production ratio of methanol produced during thermal cracking of naphtha, and means the ratio of "the number of oxygen atoms in the methanol contained in the condensed water" to "the number of oxygen atoms in the ether contained in the naphtha".

The specific method for measuring the methanol production ratio B is as described in the Examples section below.

According to the method for producing a lower olefin composition using the naphtha for producing lower olefins of the present invention, it is possible to produce a lower olefin composition that contains lower olefins and further suppresses the production of methanol. That is, a lower olefin composition having a low methanol content can be produced.

By using the method for producing a lower olefin composition of the present invention, a propylene composition containing propylene and methanol can be produced.

More specifically, by using the method for producing a lower olefin composition of the present invention, a propylene composition containing propylene and suppressing the production of methanol. That is, a propylene composition having a low methanol content, can be produced.

As mentioned above, methanol has an adverse effect on the polymerization catalyst when polymerizing lower olefins or propylene. Therefore, the method for producing a lower olefin composition of the present invention is effective when producing lower olefins such as propylene.

<Lower olefin composition>

The lower olefin composition of the present invention is a composition containing lower olefins and/or derivatives thereof, which are cracking products of the naphtha for producing lower olefins of the present invention.

The above mentioned "lower olefin" is an unsaturated hydrocarbon having 2 to 4 carbon atoms and containing one or two unsaturated bonds in one molecule. Specific examples of the lower olefin include ethylene, propylene, butenes (1-butene, 2-butene, isobutene), and butadienes (1,2-butadiene and 1,3-butadiene). Among these, at least one selected from the group consisting of ethylene, propylene, 1-butene, and 2-butene is preferred.

The "derivative thereof", i.e., the "derivative of a lower olefin" may be a compound produced during cracking of the naphtha for producing lower olefins of the present invention, or may be a compound obtained using a lower olefin that is a cracking product of the naphtha for producing lower olefins of the present invention.

The "derivative of a lower olefin" is not particularly limited, but examples thereof include the following ethylene derivatives, propylene derivatives, and butene derivatives.

a) Ethylene derivatives:

Ethylene oxide, ethylene glycol, ethanolamine, glycol ether, and the like obtained by oxidation reaction of ethylene

Vinyl chloride monomer, 1,1,1-trichloroethane, vinylidene chloride, polyvinyl chloride, and the like obtained by chlorination of ethylene

α-Olefins obtained by polymerization of ethylene, and higher alcohols obtained by oxo reaction and subsequent hydrogenation reaction using the α-olefins as raw materials, and the like

Low to high density polyethylene, and the like obtained by polymerization of ethylene

Vinyl acetate, and the like obtained by reaction of ethylene with acetic acid

Acetaldehyde and its derivative obtained by the Wacker reaction of ethylene such as ethyl acetate, and the like

b) Propylene derivatives:

Acrylonitrile, and the like obtained by ammoxidation of propylene,

Acrolein, acrylic acid, acrylic acid esters, and the like obtained by selective oxidation of propylene

Oxo alcohols such as normal butyraldehyde, 2-ethylhexanol, and the like obtained by oxo reaction of propylene

Polypropylene, and the like obtained by polymerization of propylene

Propylene oxide and propylene glycol obtained by selective oxidation of propylene, and isopropyl alcohol obtained by hydration of propylene, and the like

Acetone obtained by the Wacker reaction of propylene, methyl isobutyl ketone and acetone cyanohydrin obtained from acetone, methyl methacrylate obtained from acetone cyanohydrin, and the like.

c) Butene derivatives:

Butadiene obtained by oxidative dehydrogenation of butene

1,4-butanediol obtained through acetoxylation, hydrogenation, and hydrolysis of butadiene, γ-butyrolactone, pyrrolidones such as N-methylpyrrolidone, and the like obtained using this as a raw material

Tetrahydrofuran, polytetramethylene glycol, and the like obtained by dehydration of pyrrolidones

Various synthetic rubbers obtained using butadiene

By using the naphtha for producing lower olefins of the present invention, it is possible to produce a lower olefin composition that contains lower olefins and further suppresses the generation of methanol, that is, has a low methanol content, for the reasons described above.

In the lower olefin composition of the present invention, the ether contained in the naphtha for producing lower olefins is the asymmetric ether having the asymmetric structure with respect to the ether oxygen atom.

In the lower olefin composition of the present invention, the ether contained in the naphtha for producing lower olefins is preferably monoether having only one ether oxygen atom in the molecule, since this can more effectively reduce the content of produced methanol in the obtained lower olefins.

In the lower olefin composition of the present invention, the ether contained in the naphtha for producing lower olefins is preferably ether wherein one of the two carbon atoms bonded to the ether oxygen atom is a carbon atom of a methyl group, such as 2-methoxybutane, methoxycyclopentane, and 1-methoxypropane, since this can more effectively reduce the content of produced methanol in the obtained lower olefins.

When the ether is an ether wherein one of the two carbon atoms bonded to the ether oxygen atom is a carbon atom of a methyl group, the lower olefin composition of the present invention contains methanol.

Although the content of the lower olefin in the lower olefin composition is not particularly limited, it is preferably 70% by mass or more, more preferably 80% by mass or more, even more preferably 90% by mass or more, particularly preferably 95% by mass or more, and especially preferably 98% by mass or more, based on 100% of the total mass of the lower olefin composition. The content of the lower olefin in the lower olefin composition may be 100% by mass.

Although the content of methanol in the lower olefin composition is not particularly limited, it is preferably 10,000 ppm by mass or less, more preferably 1,000 ppm by mass or less, even more preferably 100 ppm by mass or less, particularly preferably 10 ppm by mass or less, especially preferably 5 ppm by mass or less, and most preferably 1 ppm by mass or less, based on the total mass of the lower olefin composition.

The content of methanol in the lower olefin can be measured by gas chromatography.

Such lower olefin composition can be obtained by cracking the naphtha for producing lower olefins of the present invention.

<Polyolefin-based polymer>

The polyolefin-based polymer of the present invention is a polyolefin-based polymer obtained by polymerizing the lower olefin and/or the derivative thereof contained in the lower olefin composition of the present invention using a known polymerization method. The polyolefin-based polymer of the present invention contains repeating units derived from olefins (hereinafter referred to as "lower olefin units") or repeating units derived from their derivatives (hereinafter referred to as "lower olefin derivative units").

The polyolefin-based polymer of the present invention may be a polymer containing only lower olefin units, a polymer containing lower olefin units and lower olefin derivative units, or a polymer containing only lower olefin derivative units.

The "repeating units" mentioned above refer to units formed directly by the polymerization reaction of lower olefins and/or their derivatives, and may be units wherein a part of the units is converted to a different structure by processing the polymer.

The polyolefin-based polymer of the present invention may be a polyolefin-based polymer obtained by polymerizing the lower olefin composition of the present invention from which methanol has been removed by a known methanol separation method such as distillation.

Alternatively, the polyolefin-based polymer of the present invention may be a polyolefin-based polymer obtained by polymerizing the lower olefin composition of the present invention as is. In this case, since the methanol content in the lower olefin composition is low for the reasons described above, the performance of the catalyst used in polymerizing the lower olefin is not substantially impaired by methanol, and the obtained polyolefin-based polymer is excellent in quality from the viewpoints of molecular weight distribution, impurities, and the like.

Examples

The present invention will be described in more detail below with the following Experiments in lieu of Examples and Comparative Experiments. These are merely for the purpose of explanation and do not limit the present invention in any way.

The compounds used in the Experiments and Comparative Experiments are as follows.

2-Methoxybutane (manufactured by Tokyo Chemical Industry Co., Ltd.)

Methoxycyclopentane (manufactured by Tokyo Chemical Industry Co., Ltd.)

1-Methoxypropane (manufactured by Tokyo Chemical Industry Co., Ltd.)

Dimethyl ether (manufactured by Koike Chemical Co., Ltd.)

Diethyl ether (manufactured by Tokyo Chemical Industry Co., Ltd.)

Diisopropyl ether (manufactured by Tokyo Chemical Industry Co., Ltd.)

Dipropyl ether (manufactured by Tokyo Chemical Industry Co., Ltd.)

<Evaluation method>

(1) Measurement of the content ratio of the hydrocarbons having 7 or more carbon atoms in naphtha

The content ratio of the hydrocarbons having 7 or more carbon atoms in naphtha was measured using a gas chromatograph device (GC device) under the following conditions.

<GC measurement conditions>

GC device: GC-2010 Plus (device name, manufactured by Shimadzu Corporation)

Detector: Hydrogen flame ionization detector (FID)

Carrier gas: Nitrogen (flow rate 0.8 ml/min)

Column: Capillary column CBP1-M50-025 (manufactured by Shimadzu Corporation, size: 0.22 mm diameter x 50 m, film thickness: 0.25 μm)

Column temperature: 0°C (holding time 5 min) → heating at 3°C/min → 200°C (no holding time) → heating at 10°C/min → 250°C (holding time 10 min)

Inlet temperature: 250°C

Detector temperature: 250°C

Sample volume: 0.5 μL (split ratio: 1/60)

Quantitative method: Absolute calibration curve method

Naphtha was used as the sample for GC measurement without pretreatment.

A calibration curve for calculating the content ratio of hydrocarbons from the peak areas of the gas chromatogram was prepared using, as standard solutions, compounds previously separated and purified from naphtha, commercially available compounds, and previously synthesized compounds that were accurately weighed and diluted as the respective components of the hydrocarbons having 1 to 15 carbon atoms identified from the gas chromatogram.

As an example of a gas chromatogram of naphtha, Table A shows the retention time (minutes), type, and carbon number of each hydrocarbon component contained in naphtha identified from the gas chromatogram for the mixture of petroleum-derived naphtha and biomaterial-derived naphtha used in Experiment C-1.

As shown in Table A, all of the hydrocarbon components were observed between retention times of 3.570 to 39.021 minutes, and the carbon numbers of the hydrocarbons were in the range of 3 to 10.

Here, the naphtha mixture of Experiment C-1 was used as an example for explanation, and the results were shown in Table A. However, the type and number of carbon atoms of each hydrocarbon component contained in naphtha varies depending on the origin, production area, or production conditions of the naphtha, and the like.

[Table A]

| Retention time (min) | Hydrocarbon components in naphtha | Carbon number |
|---|---|---|
| 3.570 | Normal propane | 3 |
| 3.927 | Isobutane | 4 |
| 4.259 | Normal butane | 4 |
| 5.684 | Isopentane | 5 |
| 6.324 | 2-Methyl-1-butene | 5 |
| 6.524 | Normal pentane | 5 |

(continued)

| Retention time (min) | Hydrocarbon components in naphtha | Carbon number |
|---|---|---|
| 6.798 | 2-Pentene | 5 |
| 7.296 | 2-Methyl-2-butene | 5 |
| 7.848 | 2,2-Dimethylbutane | 6 |
| 9.150 | Cyclopentane | 5 |
| 9.372 | 2,3-Dimethylbutane | 6 |
| 9.730 | 2-Methylpentane | 6 |
| 10.522 | 3-Methylpentane | 6 |
| 11.651 | Normal hexane | 6 |
| 13.158 | Methylcyclopentane | 6 |
| 13.601 | 2,4-Dimethylpentane | 7 |
| 13.790 | 2,2-Dimethylpentane | 7 |
| 14.791 | Benzene | 6 |
| 15.221 | 3,3-Dimethylpentane | 7 |
| 15.309 | Cyclohexane | 6 |
| 16.340 | 2-Methylhexane | 7 |
| 16.929 | 3-Methylhexane | 7 |
| 17.153 | trans-1,3-Dimethylcyclopentane | 7 |
| 17.347 | cis-1,3-Dimethylcyclopentane | 7 |
| 17.552 | 1,2-Dimethylcyclopentane | 7 |
| 18.757 | Normal heptane | 7 |
| 19.839 | Methylcyclohexane | 7 |
| 20.136 | 1,1,3-Trimethylcyclopentane | 8 |
| 20.733 | Ethylcyclopentane | 7 |
| 21.035 | 2,5-Dimethylhexane | 8 |
| 21.140 | 2,4-Dimethylhexane | 8 |
| 21.385 | 1,2,4-Trimethylcyclopentane | 8 |
| 21.588 | 3,3-Dimethylhexane | 8 |
| 21.939 | 1,2,3-Trimethylcyclopentane | 8 |
| 22.135 | 2,3,3-Trimethylpentane | 8 |
| 22.363 | Toluene | 7 |
| 23.042 | 1,1,2-Trimethylcyclopentane | 8 |
| 23.515 | 2-Methylheptane | 8 |
| 23.601 | 4-Methylheptane | 8 |
| 23.934 | 1,3-Dimethylhexane | 8 |
| 24.043 | 3-Methylheptane | 8 |
| 24.422 | 1,1-Dimethylcyclohexane | 8 |
| 24.782 | trans-1-Ethyl-3-methylcyclopentane | 8 |
| 24.926 | cis-1-Ethyl-3-methylcyclopentane | 8 |
| 25.011 | 1-Ethyl-2-methylcyclopentane | 8 |

(continued)

| Retention time (min) | Hydrocarbon components in naphtha | Carbon number |
| --- | --- | --- |
| 25.231 | 1-Ethyl-1-methylcyclopentane | 8 |
| 25.943 | Normal octane | 8 |
| 26.281 | Isopropylcyclopentane | 8 |
| 27.030 | Methylcycloheptane | 8 |
| 27.567 | 2,3,5-Trimethylhexane | 9 |
| 27.762 | Ethylcyclohexane | 8 |
| 28.028 | 2,6-Dimethylheptane | 9 |
| 28.156 | 1,1,4-Trimethylcyclohexane | 8 |
| 28.440 | 2,5-Dimethylheptane | 9 |
| 29.032 | Ethylbenzene | 8 |
| 29.193 | 1,3,5-Trimethylcyclohexane | 9 |
| 29.619 | m-Xylene | 8 |
| 29.690 | p-Xylene | 8 |
| 29.938 | Cyclononane | 9 |
| 30.167 | 4-Ethylheptane | 9 |
| 30.380 | 2-Methyloctane | 9 |
| 30.798 | 1,1,3-Trimethylhexane | 9 |
| 30.860 | 3-Methyloctane | 9 |
| 31.097 | o-Xylene | 8 |
| 31.586 | 1-Ethyl-4-methylcyclohexane | 9 |
| 31.754 | 1-Ethyl-3-methylcyclohexane | 9 |
| 33.359 | Propylbenzene | 9 |
| 33.373 | Isopropylbenzene | 9 |
| 34.362 | 2,5-Dimethyloctane | 10 |
| 34.952 | 3,6-Dimethyloctane | 10 |
| 35.236 | 1-Ethyl-2-methylbenzene | 9 |
| 35.628 | 3-Ethyl-1-methylbenzene | 9 |
| 35.757 | 4-Ethyl-1-methylbenzene | 9 |
| 36.169 | 1,3,5-Trimethylbenzene | 9 |
| 36.794 | Cyclodecane | 10 |
| 36.819 | 2-Eethylstyrene, 3-Methylstyrene | 9 |
| 37.697 | 1,2,4-Trimethylbenzene | 9 |
| 39.021 | Normal decane | 10 |

The content ratio of the hydrocarbons having 7 or more carbon atoms in the naphtha was calculated according to the following procedure.

First, the peak area of each hydrocarbon component having 1 to 15 carbon atoms was read from the peaks observed between the retention time of 1 to 100 minutes in the gas chromatogram obtained using the above GC measurement conditions, and the content ratio (unit:% by mass) of each component was calculated using the absolute calibration curve method.

Next, the total content ratio of each hydrocarbon component having 1 to 15 carbon atoms was set as B1, and the total content ratio of each hydrocarbon component having 7 to 15 carbon atoms was set as A1, and the content ratio of hydrocarbons having 7 or more carbon atoms in naphtha was calculated according to the following formula.

Content ratio of the hydrocarbons having 7 or more carbon atoms (% by mass) = A1/B1 $\times$ 100

A1: Total content ratio of each hydrocarbon component having 7 to 15 carbon atoms (% by mass)

B1: Total content ratio of each hydrocarbon component having 1 to 15 carbon atoms (% by mass)

(2) Calculation of the average molecular weight of hydrocarbons in naphtha

The average molecular weight of hydrocarbons in naphtha was calculated from the following formula using B1 calculated using the measurement method of "(1) the content ratio of the hydrocarbons having 7 or more carbon atoms in naphtha" described above, the content ratio of each hydrocarbon component having 1 to 15 carbon atoms Sn, and the relative molecular mass Mn.

Average molecular weight of the hydrocarbons in the naphtha = (S1 $\times$ M1 + S2 $\times$ M2 + .....S15 $\times$ M15)/B1

Sn (n is an integer from 1 to 15): Content ratio (% by mass) of the hydrocarbons having n carbon atoms

Mn (n is an integer from 1 to 15): Relative molecular mass of the hydrocarbons having n carbon atoms

B1: Total content ratio of each hydrocarbon component having 1 to 15 carbon atoms (% by mass)

The relative molecular mass is the ratio of the mass of one molecule of a substance to the unified atomic mass unit, and refers to the sum of the atomic mass contained in a molecule.

A hydrocarbon having a certain number of carbon atoms may be composed of multiple hydrocarbon components having different relative molecular mass. For example, a hydrocarbon having 5 carbon atoms is composed of six types of hydrocarbons: isopentane, 2-methyl-1-butene, normal pentane, 2-pentene, 2-methyl-2-butene, and cyclopentane.

(3) Analysis of the content of the sulfur-containing compounds in naphtha

As the sulfur content of the sulfur-containing compounds in naphtha, the content of the sulfur-containing compounds converted into sulfur atoms (content in terms of sulfur atoms) was measured by the inductively coupled plasma optical emission spectrometry (ICP-OES).

First, a sample for ICP-OES measurement was prepared according to the following procedure.

After mixing 1 ml of naphtha and 8 ml of nitric acid in a Teflon container, the mixture was decomposed by heating under the following conditions using a microwave decomposition device (device name: MultiwavePRO, manufactured by Anton Paar), and then the volume was adjusted to 25 ml with ultrapure water, and this was used as the sample for ICP-OES measurement.

Microwave decomposition conditions: Ramp (500 W/10 min) → Hold (500 W/10 min) → Ramp (1300 W/10 min) → Hold (1300 W/30 min)

The concentration (unit: ppm by mass) of sulfur atoms in naphtha was measured using an ICP-OES (device name: iCAP6500, manufactured by Thermo Fisher, wavelength: S = 180.731 nm) for the measurement sample, and this was taken as the content of sulfur-containing compounds converted into sulfur atoms.

The calibration curve was created using the absolute calibration curve method using Sulfur Standard for ICP (product name, manufactured by Aldrich).

(4) Calculation method of the $\Delta E$

The absolute value $\Delta E$ [unit: e] of the difference in charge between the two carbon atoms bonded to the ether oxygen atom in the ether used in the Experiment or Comparative Experiment was calculated according to the following procedure.

The charge values of the two carbon atoms were calculated by the density functional theory method (DFT) calculations on the molecular structure of the ether. For the DFT calculation conditions, def-TZVP was used as the basis set, and the COSMO solvation model (COnductor-like Screening MOdel) was used as the solvent effect. The Mulliken's charge density analysis method (Population Analysis) was used as the analysis method.

Next, for the two carbon atoms bonded to the oxygen atom of the ether bond in the ether, the absolute value $\Delta E$ ($\Delta E = |E1 - E2|$) (unit: e) of the difference between the charge E1 of one carbon atom and the charge E2 of another carbon atom was calculated.

"e" means the elementary charge, and e = 1.602176634 x $10^{-19}$ [unit: C]. For example, when the $\Delta E$ is 0.05 [unit: e], this can be expressed in SI units as $\Delta E = 0.05 \times 1.602176634 \times 10^{-19}$ [unit: C].

To calculate the $\Delta E$, the quantum chemistry calculation software "TURBOMOLE ver. 7.2" (manufactured by TURBOMOLE) and the graphical user interface for TURBOMOLE "TmoleX ver. 4.4.1" (manufactured by TURBOMOLE) were used.

(5) Calculation of the methanol production ratio B

Methanol produced by the thermal cracking of naphtha is substantially contained in the condensed water obtained in Experiment or Comparative Experiment, and is not contained in the gas component or oil content. For this reason, the methanol production ratio B of the condensed water obtained in Experiment or Comparative Experiment was measured under the following conditions using a gas chromatography mass spectrometry measuring device (GC/MS device) (device name: GCMS-QP2010Ultra, manufactured by Shimadzu Corporation).

It was confirmed in advance that methanol was not produced from the blank naphtha used in Experiments and Comparative Experiments.

<GC/MS measurement conditions>

Carrier gas: Helium, linear velocity 40 cm/sec

Column: SUPELCOWAX-10 (manufactured by Supelco, inner diameter of 0.32 mm x length of 60 m x film thickness of 0.25 $\mu$m)

Temperature (heating conditions): 50°C (holding time 5 min) $\rightarrow$ heating at 20°C/min $\rightarrow$ 200°C (holding time 2.5 min)

Inlet temperature: 200°C

MS interface temperature: 200°C

Ion source temperature: 200°C

Sample volume: 0.5 $\mu$L

Split ratio: 1:5

Measurement mode: SIM (m/z = 31)

The amount of the methanol produced as a thermal cracking product was quantified from the condensed water obtained in Experiment or Comparative Experiment, and the methanol production ratio B to the added ether was calculated from the following formula based on a calibration curve previously prepared using a standard solution of methanol with a known concentration.

[Methanol production ratio B] = [Number of oxygen atoms in methanol in the condensed water]/[Number of oxygen atoms in the ether added to blank naphtha]

In other words, when the methanol production ratio B is 1.00, it means that all the added ether was quantified as methanol.

The number of oxygen atoms contained in the added ether is one oxygen atom per molecule of the ether compound.

6) Measurement of the lower olefins yield

The lower olefins yield when the raw naphtha was thermally cracked was measured using a naphtha flow evaluation device equipped with a SUS310S reaction tube, using the following method.

The "lower olefin" refers to ethylene, propylene, butenes (1-butene, 2-butene, and isobutene), and butadienes (1,2-butadiene and 1,3-butadiene) as mentioned above.

The naphtha and steam from Experiments and Comparative Experiments described below were supplied to the reaction tube (inner diameter of 4 mm x outer diameter of 6 mm) from the bottom of the reaction tube, and the naphtha was thermal cracked under conditions of a reaction temperature of 810°C, a reaction pressure of 0.1 MPaG, a residence time of 0.6 seconds, and a steam/naphtha mass ratio (S/O ratio) of 0.4. Then, the product was extracted from the top of the reaction tube. The resulting thermal cracking product was quenched at 5°C and subjected to gas-liquid separation using a gas-liquid separator under conditions of 0.1 MPaG and 5°C to obtain separated gas and separated liquid.

The separated gas obtained was subjected to quantitative analysis of the amounts of hydrogen, carbon monoxide, carbon dioxide, and hydrocarbons such as methane, ethane, ethylene, propane, propylene, propadiene, isobutane, normal butane, allene, 1-butene, 2-butene, isobutene, 1,2-butadiene, 1,3-butadiene, methylacetylene, isopentane, normal pentane, 1-pentene, 2-pentene, cyclopentane, 2-methyl-1-butene, 2-methyl-2-butene, cyclopentene, isoprene, pentadiene, cyclopentadiene, benzene, toluene, xylene, normal hexane, ethylbenzene, styrene, normal heptane, and normal octane (hereinafter, referred to as "hydrocarbons"), which were the naphtha thermal cracking products, using individual gas chromatograph measuring devices.

Specifically, a gas chromatograph measuring device (model number: GC-8A, manufactured by Shimadzu Corporation) was used to quantify hydrogen.

A gas chromatograph measuring device (model number: GC-2014, manufactured by Shimadzu Corporation) was used to quantify carbon monoxide, carbon dioxide, and the above mentioned hydrocarbons.

The quantitative values of hydrogen, carbon monoxide, carbon dioxide, hydrocarbons, and lower olefins measured in this manner were designated X (hydrogen), X (carbon monoxide), X (carbon dioxide), X (hydrocarbons), and X (lower olefins) (unit: g), respectively.

The lower olefins contained in the separated liquid on the oil side as thermal cracking products of the naphtha were quantified using a gas chromatograph measuring device (model number: GC-2014, manufactured by Shimadzu Corporation), and the quantitative value was designated Y (lower olefins) (unit: g).

The above mentioned hydrocarbons and hydrocarbons having boiling points of 150°C or higher and 280°C or lower were quantified, and these quantitative values were combined to give Y (hydrocarbon compounds) (unit: g).

The lower olefins yield (unit:% by mass) was calculated according to the following formula using X (hydrogen), X (carbon monoxide), X (carbon dioxide), X (lower olefins), X (hydrocarbons), Y (lower olefins), and Y (hydrocarbon compounds) measured by the above mentioned method between 60 and 420 minutes after the start of the thermal cracking of the naphtha.

Lower olefins yield (unit: % by mass) = (X (lower olefins) + Y (lower olefins))/(X (hydrogen) + X (carbon monoxide), X (carbon dioxide) + X (hydrocarbons) + Y (hydrocarbon compounds)) $\times$ 100

[Comparative Experiment A-1-1]

2-Methoxybutane was added to the petroleum-derived naphtha used as the raw material (open spec naphtha from Saudi Arabia, content ratio of hydrocarbons having 7 or more carbon atoms: 13.1% by mass, average molecular weight of hydrocarbons: 79.2 g/mol, specific gravity: 0.6636 g/cm$^3$, content of sulfur-containing compounds (in terms of sulfur atoms): 190 ppm by mass) so that the content of oxygen atoms derived from ether was 50 ppm by mass, and then the mixture was thermally decomposed in the presence of steam using a thermal cracking furnace under the following thermal cracking conditions. The obtained thermal cracking product was quenched at 5°C and separated into gas and liquid under conditions of 0.1MPa (gauge pressure) and 5°C using a gas-liquid separator to obtain gas components and separated liquid.

Furthermore, the separated liquid was separated into oil and condensed water using a separatory funnel under conditions of atmospheric pressure and room temperature.

<thermal cracking conditions>

Naphtha flow rate: 83.1g/hr

Steam/naphtha mass ratio: 0.4

Residence time: 0.6 seconds

Thermal cracking temperature: 810°C

Thermal cracking pressure: 0.1MPa (gauge pressure)

The methanol generation ratio B of the condensed water was measured using the above mentioned method. The methanol generation ratio B was shown in Table 1 together with the value of $\Delta E$. The relationship between the $\Delta E$ and methanol generation ratio B was also shown in Fig. 1.

[Comparative Experiments A-1-2 to A-1-3]

Evaluation was carried out under the same conditions as Comparative Experiment A-1-1, except that the content in terms of ether oxygen atoms was changed as shown in Table 1. The evaluation results were shown in Table 1.

[Comparative Experiment A-0]

Evaluation was carried out under the same conditions as Comparative Experiment A-1-1, except that 2-methoxybutane was not used. The evaluation results were shown in Table 1.

[Comparative Experiments A-2 to A-7]

Evaluation was carried out under the same conditions as Comparative Experiment A-1, except that the type of ether and the content of the ether in terms of ether oxygen atoms were changed as shown in Table 1. The evaluation results were shown in Table 1.
Comparative Experiment A-1 includes Comparative Experiment A-1-1 to Comparative Experiment A-1-3 shown in Table 1.
Comparative Experiment A-2 includes Comparative Experiment A-2-1 to Comparative Experiment A-2-3 in Table 1.
Comparative Experiment A-3 includes Comparative Experiment A-3-1 to Comparative Experiment A-3-3 in Table 1.
In the following Tables 1 to 3, the content of the added ether in terms of ether oxygen atoms is referred to as "oxygen atom concentration."

[Table 1]

<Table 1 >

| | Naphtha | Added ether | $\Delta E$ of ether [unit: e] | Oxygen atom concentration [ppm by mass] | Methanol production ratio B [-] | Low olefins yield [% by mass] |
|---|---|---|---|---|---|---|
| Comparative Experiment A-0 | Petroleum-derived naphtha | None | - | - | 0.00 | 49.8 |
| Comparative Experiment A-1-1 | Petroleum-derived naphtha | 2-Methoxybutane | 0.181 | 50 | 0.23 | 49.8 |
| Comparative Experiment A-1-2 | Petroleum-derived naphtha | 2-Methoxybutane | 0.181 | 500 | 0.23 | - |

(continued)

| | Naphtha | Added ether | ΔE of ether [unit: e] | Oxygen atom concentration [ppm by mass] | Methanol production ratio B [-] | Low olefins yield [% by mass] |
|---|---|---|---|---|---|---|
| Comparative Experiment A-1-3 | Petroleum-derived naphtha | 2-Methoxybutane | 0.181 | 5000 | 0.22 | 51.6 |
| Comparative Experiment A-2-1 | Petroleum-derived naphtha | Methoxycyclopentane | 0.151 | 50 | 0.16 | 51.2 |
| Comparative Experiment A-2-2 | Petroleum-derived naphtha | Methoxycyclopentane | 0.151 | 500 | 0.16 | 51.2 |
| Comparative Experiment A-2-3 | Petroleum-derived naphtha | Methoxycyclopentane | 0.151 | 5000 | 0.17 | 50.9 |
| Comparative Experiment A-3-1 | Petroleum-derived naphtha | 1-Methoxypropane | 0.084 | 50 | 0.10 | 50.5 |
| Comparative Experiment A-3-2 | Petroleum-derived naphtha | 1-Methoxypropane | 0.084 | 500 | 0.11 | - |
| Comparative Experiment A-3-3 | Petroleum-derived naphtha | 1-Methoxypropane | 0.084 | 5000 | 0.10 | - |
| Comparative Experiment A-4 | Petroleum-derived naphtha | Dimethylether | 0.004 | 50 | 0.01 | 51.3 |
| Comparative Experiment A-5 | Petroleum-derived naphtha | Dimethylether | 0.001 | 50 | 0.01 | - |
| Comparative Experiment A-6 | Petroleum-derived naphtha | Diisopropylether | 0.010 | 50 | 0.00 | 50.0 |
| Comparative Experiment A-7 | Petroleum-derived naphtha | Di Propylether | 0.000 | 50 | 0.00 | 49.6 |

[Experiments B-1 to B-3, Comparative Experiments B-4 and B-7]

The evaluation was carried out under the same conditions as Comparative Experiment A-1, except that a biomaterial-derived naphtha (manufactured by AltAir Paramount, content ratio of hydrocarbons having 7 or more carbon atoms: 43.9% by mass, average molecular weight of hydrocarbons: 88.3 g/mol, specific gravity: 0.6700 g/cm$^3$, content of sulfur-containing compounds (in terms of sulfur atoms): 1ppm by mass) was used instead of the petroleum-derived naphtha in Comparative Experiment A-1, and the type of ether and the content of the ether in terms of ether oxygen atoms were changed as shown in Table 2. The evaluation results were shown in Table 2.
Experiment B-2 includes Experiment B-2-1 to Experiment B-2-3 shown in Table 2.

[Experiment B-0]

The evaluation was carried out under the same conditions as Experiment B-1, except that 2-methoxybutane was not used. The evaluation results were shown in Table 2.

[Table 2]

<Table 2 >

| | Naphtha | Added ether | ΔE of ether [unit: e] | Oxygen atom concentration [ppm by mass] | Methanol production ratio B [-] | Low olefins yield [% by mass] |
|---|---|---|---|---|---|---|
| Experiment B-0 | Bio-derived naphtha | None | - | - | 0.00 | 56.6 |
| Experiment B-1 | Bio-derived naphtha | 2-Methoxybutane | 0.181 | 50 | 0.18 | 55.2 |
| Experiment B-2-1 | Bio-derived naphtha | Methoxycyclopentane | 0.151 | 50 | 0.13 | 56.1 |
| Experiment B-2-2 | Bio-derived naphtha | Methoxycyclopentane | 0.151 | 500 | 0.12 | 55.2 |
| Experiment B-2-3 | Bio-derived naphtha | Methoxycyclopentane | 0.151 | 5000 | 0.14 | 56.0 |
| Experiment B-3 | Bio-derived naphtha | 1-Methoxypropane | 0.084 | 50 | 0.09 | 56.5 |
| Comparative Experiment B-4 | Bio-derived naphtha | Dimethylether | 0.004 | 50 | 0.00 | 56.2 |
| Comparative Experiment B-7 | Bio-derived naphtha | Dipropylether | 0.000 | 50 | 0.00 | 56.8 |

[Experiment C-1]

The evaluation was carried out under the same conditions as Comparative Experiment A-1, except that a mixture of the petroleum-derived naphtha and the bio raw material-derived naphtha (mass ratio 1:1) (content ratio of hydrocarbons having 7 or more carbon atoms: 30.6% by mass, average molecular weight of hydrocarbons: 84.14 g/mol, specific gravity: 0.6667 g/cm$^3$, content of sulfur-containing compounds (in terms of sulfur atoms): 100ppm by mass) was used instead of the petroleum-derived naphtha in Comparative Experiment A-1, and the type of ether and the content of the ether in terms of ether oxygen atoms were changed as shown in Table 3. The evaluation results were shown in Table 3.

[Experiment C-0]

The evaluation was carried out under the same conditions as Experiment C-1, except that 2-methoxybutane was not used. The evaluation results were shown in Table 3.

[Table 3]

< Table 3 >

| | Comparative Experiment C-0 | Experiment C-1 |
|---|---|---|
| Naphtha | Mixture of petroleum-derived naphtha and bio-derived naphtha (mass ratio 1:1) | Mixture of petroleum-derived naphtha and bio-derived naphtha (mass ratio 1:1) |
| Added ether | None | 2-Methoxybutane |
| Content ratio of C7 or higher hydrocarbons [% by mass] | 30.6 | 30.6 |
| Average molecular weight of hydrocarbons [g/mol] | 84.1 | 84.1 |
| Specific gravity [g/cm$^3$] | 0.6667 | 0.6667 |
| ∆E of ether [Unit: e] | - | 0.181 |
| Oxygen atom concentration [ppm by mass] | - | 50 |
| Methanol production ratio B [-] | 0.00 | 0.20 |
| Ethylene yield [% by mass] | 27.0 | 27.0 |
| Propylene yield [% by mass] | 16.7 | 16.7 |
| Butene yield [% by mass] | 5.8 | 5.8 |
| Butadiene yield [% by mass] | 3.1 | 3.1 |
| Low olefins yield [% by mass] | 52.6 | 52.6 |

In Fig. 1, the results of Experiments B-1, B-2-1, and B-3, and Experiment C-1, as well as Comparative Experiments A-1-1, A-2-1, A-3-1, A-4, A-7, B-4, and B-7 were plotted.

Comparing Experiments B-1 to B-3 and Comparative Experiments B-4 and B-7 with Comparative Experiments A-1 to A-7, it can be seen that the naphtha derived from bio raw materials has a lower methanol production ratio B and is less likely to produce methanol than the naphtha derived from a fossil fuels.

The reason for this is not clear, however it is presumed to be as follows.

The naphtha derived from bio raw material generally contains hydrocarbons having large molecular weight compared to the naphtha derived from a fossil fuel, so the naphtha derived from bio raw materials tends to have lower partial pressure of the hydrocarbons. As a result, in the naphtha derived from bio raw materials, the side reaction for producing methanol from the asymmetric ethers is suppressed. Therefore, compared to the naphtha derived from a fossil fuel, methanol is less likely to be produced even when thermal cracked under thermal cracking conditions.

Furthermore, in all naphtha, it can be seen that for any naphtha, ethers having a ∆E of 0.05 or more tend to produce more methanol as the ∆E value increases.

Comparing Experiments B-1 to B-3 with Comparative Experiments B-4 and B-7, as well as comparing Comparative Experiments A-1 to A-7, it can be seen that there is a correlation between the ∆E value of the ether and the methanol production ratio B, and that ethers that have an asymmetric structure with respect to an ether oxygen atom have a high methanol production ratio B and are more likely to produce methanol. Furthermore, it can be seen that ethers having a ∆E of 0.05 or more tend to produce more methanol as the ∆E value increases.

As shown in Experiment B-2 in Table 2, when the concentration of ether having an asymmetric structure with respect to an oxygen atom constituting an ether bond was 20,000 ppm by mass or less in terms of ether oxygen atoms, the methanol production ratio was approximately the same when the concentration of the ether was 50 ppm by mass, 500 ppm by mass, and 5,000 ppm by mass, in terms of ether oxygen atoms.

The reason for this is presumed to be as follows.

Since ethers having a asymmetric structure have a large ∆E, there is a large bias in the charge between the two carbon atoms bonded to the ether oxygen atom, and they are easily thermal decomposed under thermal cracking conditions to produce methanol.

The detals of the lower olefins yields (unit:% by mass) in Comparative Experiment A-1-1, Experiment B-1, and Experiment C-1 were shown in Table 4.

In this evaluation, "lower olefins" refers to ethylene, propylene, butenes (1-butene, 2-butene, and isobutene), and butadienes (1,2-butadiene and 1,3-butadiene), as mentioned above.

[Table 4]

< Table 4 >

| | Comparative Experiment A-1-1 | Experiment C-1 | Experiment B-1 |
|---|---|---|---|
| Naphtha | Petroleum-derived naphtha | Mixture of petroleum-derived naphtha and bio-derived naphtha (mass ratio 1:1) | Bio-derived naphtha |
| Added ether | 2-Methoxybutane | 2-Methoxybutane | 2-Methoxybutane |
| Content ratio of C7 or higher hydrocarbons [% by mass] | 13.1 | 30.6 | 43.9 |
| Average molecular weight of hydrocarbons [g/mol] | 79.2 | 84.1 | 88.3 |
| Specific gravity [g/cm$^3$] | 0.6636 | 0.6667 | 0.6700 |
| ΔE of ether [Unit: e] | 0.181 | 0.181 | 0.181 |
| Oxygen atom concentration [ppm by mass] | 50 | 50 | 50 |
| Methanol production ratio B [-] | 0.23 | 0.20 | 0.18 |
| Ethylene yield [% by mass] | 25.4 | 27.0 | 29.3 |
| Propylene yield [% by mass] | 16.0 | 16.7 | 17.5 |
| Butene yield [% by mass] | 5.5 | 5.8 | 6.2 |
| Butadiene yield [% by mass] | 2.9 | 3.1 | 3.6 |
| Low olefins yield [% by mass] | 49.8 | 52.6 | 55.2 |

From Table 4, it can be seen that the higher the content of the biomaterial-derived naphtha in the naphtha, the higher the yield of each lower olefin.

Therefore, it can be seen that, by using naphtha as the raw material, for example, that contains naphtha derived from bio raw material, in which the content ratio of hydrocarbons having 7 or more carbon atoms is a predetermined value or more, and the concentration of ether having an asymmetric structure with respect to an ether oxygen atom, in terms of ether oxygen atoms, is set to a predetermined value or less, it is possible to suppress the methanol production concentration and produce lower olefins having high commercial value.

[Reference Experiments D-1 to D-3]

A mixture of the petroleum-derived naphtha and the bio raw material-derived naphtha (mass ratio 1:1) (content ratio of hydrocarbons having 7 or more carbon atoms: 32.1% by mass, average molecular weight of hydrocarbons: 85.0 g/mol, specific gravity: 0.6712 g/cm$^3$) was obtained in the same manner as in Comparative Experiment C-1, except that in the mixture of petroleum-derived naphtha and the biomaterial-derived naphtha (mass ratio 1:1) in Comparative Experiment C-1, the petroleum-derived naphtha B (Japanese naphtha, content ratio of hydrocarbons having 7 or more carbon atoms: 16.3% by mass, average molecular weight of hydrocarbons: 80.4 g/mol, specific gravity: 0.6656 g/cm$^3$) was used instead of the above mentioned petroleum-derived naphtha, the biomaterial-derived naphtha B (manufactured by AltAir Paramount, content ratio of hydrocarbons having 7 or more carbon atoms: 38.6% by mass, average molecular weight of hydrocarbons: 88.6 g/mol, specific gravity: 0.671 g/cm$^3$) was used instead of the above mentioned biomaterial-derived naphtha, and the type of ether and the content of the ether in terms of ether oxygen atoms were changed as shown in Table 5.

**EP 4 574 931 A1**

[Table 5]

< Table 5 >

| | Reference Experiment D-1 | Reference Experiment D-2 | Reference Experiment D-3 |
|---|---|---|---|
| Naphtha | Mixture of petroleum -derived naphtha B and bio-derived naphtha B (mass ratio 1:1) | Mixture of petroleum -derived naphtha 8 and bio-derived naphtha B (mass ratio 1:1) | Mixture of petroleum -derived naphtha B and bio-derived naphtha B (mass ratio 1:1) |
| Added ether | 2-Methoxybutane | 2-Methoxybutane | 2-Methoxybutane |
| Content ratio of C7 or higher hydrocarbons [% by mass] | 32.1 | 32.1 | 32.1 |
| Average molecular weight of hydrocarbons [g/mol] | 85.0 | 85.0 | 85.0 |
| Specific gravity [g/cm$^3$] | 0.6692 | 0.6692 | 0.6692 |
| ΔE of ether [unit: e] | 0.181 | 0.181 | 0.181 |
| Oxygen atom concentration [ppm by mass] | 5 | 10 | 15 |

By using raw naphtha shown in Table 5, which is a mixture of petroleum-derived naphtha and the biomaterial-derived naphtha, and in which the content ratio of hydrocarbons having 7 or more carbon atoms is a predetermined value or more and the concentration of ether having an asymmetric structure with respect to an ether oxygen atom is a predetermined value or less in terms of ether oxygen atoms, it is expected that the methanol production concentration can be suppressed, the yield of low olefins can be high, and low olefins having high product value can be produced.

Although the present invention has been described in detail with reference to particular embodiments, it will be apparent to those skilled in the art that various changes may be made thereto without departing from the spirit and scope of the present invention.

The present application is based on Japanese Patent Application No. 2022-131236 filed on August 19, 2022, which is herein incorporated in its entirety by reference.

**Claims**

1. Naphtha for producing lower olefins, the naphtha containing ether and hydrocarbons having 7 or more carbon atoms,

   wherein the ether has an asymmetric structure with respect to an oxygen atom constituting an ether bond,
   a content of the hydrocarbons having 7 or more carbon atoms is 14.0% by mass or more based on 100% by mass of a total mass of the naphtha for producing lower olefins, and
   a content of the ether is 20,000 ppm by mass or less in terms of ether oxygen atoms.

2. The naphtha for producing lower olefins according to claim 1, wherein an average molecular weight of the hydrocarbons contained in the naphtha for producing lower olefins is 80.0 g/mol or more.

3. The naphtha for producing lower olefins according to claim 1, having a specific gravity of 0.6640 g/cm$^3$ or more.

4. The naphtha for producing lower olefins according to claim 1, wherein the content of the ether is 0.1 ppm by mass or more in terms of ether oxygen atoms.

5. The naphtha for producing lower olefins according to claim 1, wherein a content of sulfur-containing compounds is 180 ppm by mass or less in terms of sulfur atoms.

6. The naphtha for producing lower olefins according to claim 1, wherein the content of the hydrocarbons having 7 or more carbon atoms is 42.0% by mass or less based on 100% by mass of a total mass of the naphtha for producing lower olefins.

7. The naphtha for producing lower olefins according to claim 1, wherein an average molecular weight of the hydrocarbons contained in the naphtha for producing lower olefins is 87.0 g/mol or less.

8. The naphtha for producing lower olefins according to claim 1, having a specific gravity of 0.6695 $g/cm^3$ or less.

9. The naphtha for producing lower olefins according to claim 1, wherein a content of sulfur-containing compounds is 1 ppm by mass or more in terms of sulfur atoms.

10. The naphtha for producing lower olefins according to claim 1, wherein the naphtha for producing lower olefins includes naphtha derived from a bio raw material.

11. The naphtha for producing lower olefins according to claim 10, wherein the naphtha for producing lower olefins is naphtha derived from a bio raw material alone, or a naphtha mixture including naphtha derived from a bio raw material and naphtha derived from a fossil fuel.

12. The naphtha for producing lower olefins according to claim 10, wherein the naphtha derived from a bio raw material is naphtha derived from a non-edible biomass and/or a non-fossil fuel.

13. The naphtha for producing lower olefins according to claim 1, wherein the ether has an absolute value ∆E (∆E=|E1-E2|) of the difference between a charge E1 of one carbon atom and a charge E2 of another carbon atom, calculated by the density functional theory method for two carbon atoms bonded to an oxygen atom constituting an ether bond, of 0.05 [unit: e] or more,
wherein e means the elementary charge, and e = 1.602176634 x $10^{-19}$ [unit: C].

14. The naphtha for producing lower olefins according to claim 1, wherein the ether is monoether.

15. The naphtha for producing lower olefins according to claim 1, wherein one of two carbon atoms bonded to an oxygen atom constituting an ether bond of the ether is a carbon atom of a methyl group.

16. The naphtha for producing lower olefins according to claim 1, wherein the lower olefin is propylene.

17. A method for producing a lower olefin composition, the method comprising a step of cracking the naphtha for producing lower olefins according to any one of claims 1 to 16.

18. The method for producing a lower olefin composition according to claim 17, wherein the naphtha for producing lower olefins is cracked to produce a lower olefin composition containing lower olefins and methanol.

19. The method for producing a lower olefin composition according to claim 18, wherein the lower olefin contained in the lower olefin composition includes propylene.

20. A lower olefin composition comprising a lower olefin and/or a derivative thereof, the composition being a cracking product of the naphtha for the producing lower olefins according to any one of claims 1 to 16.

21. The lower olefin composition according to claim 20, wherein the lower olefin contained in the lower olefin composition is an unsaturated hydrocarbon having 2 to 4 carbon atoms and containing one or two unsaturated bonds in one molecule.

22. The lower olefin composition according to claim 20, further comprising methanol.

23. A polyolefin-based polymer obtained by polymerizing the lower olefin and/or the derivative thereof contained in the lower olefin composition according to claim 20.

24. A method for producing naphtha for producing lower olefins, comprising blending naphtha derived from a bio raw material and naphtha derived from a fossil fuel to obtain the naphtha for producing lower olefins according to any one of claims 1 to 16.

25. A method for determining naphtha for producing lower olefins,

comprising blending naphtha derived from a bio raw material and naphtha derived from a fossil fuel to obtain a blended naphtha,
determining that the blended naphtha, when it corresponds to the naphtha for producing lower olefins according to any one of claims 1 to 16, is acceptable as a naphtha composition to be used in producing lower olefins, and subjecting it to a thermal cracking process.

[Fig. 1]

**EP 4 574 931 A1**

## INTERNATIONAL SEARCH REPORT

| | International application No. |
|---|---|
| | **PCT/JP2023/029880** |

| A. | CLASSIFICATION OF SUBJECT MATTER |
|---|---|

*C10G 9/00*(2006.01)i; *C10G 3/00*(2006.01)i
FI:   C10G9/00; C10G3/00 Z

According to International Patent Classification (IPC) or to both national classification and IPC

| B. | FIELDS SEARCHED |
|---|---|

Minimum documentation searched (classification system followed by classification symbols)

C10G9/00; C10G3/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2017-537174 A (TOTAL RESEARCH & TECHNOLOGY FELUY) 14 December 2017 (2017-12-14) | 1-25 |
| A | JP 2008-81417 A (MITSUBISHI CHEMICALS CORP) 10 April 2008 (2008-04-10) | 1-25 |
| A | WO 2011/012439 A1 (TOTAL PETROCHEMICALS RESEARCH FELUY) 03 February 2011 (2011-02-03) | 1-25 |

☐ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **26 September 2023** | **10 October 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

29

# EP 4 574 931 A1

## INTERNATIONAL SEARCH REPORT
### Information on patent family members

International application No.

**PCT/JP2023/029880**

| Patent document cited in search report | | | Publication date (day/month/year) | Patent family member(s) | | | Publication date (day/month/year) |
|---|---|---|---|---|---|---|---|
| JP | 2017-537174 | A | 14 December 2017 | US | 2017/0298280 | A1 | |
| | | | | WO | 2016/058953 | A1 | |
| | | | | EP | 3207105 | A1 | |
| | | | | KR | 10-2017-0068454 | A | |
| JP | 2008-81417 | A | 10 April 2008 | (Family: none) | | | |
| WO | 2011/012439 | A1 | 03 February 2011 | US | 2012/0157728 | A1 | |
| | | | | EP | 2290035 | A1 | |
| | | | | KR | 10-2012-0032540 | A | |
| | | | | CN | 102549112 | A | |

Form PCT/ISA/210 (patent family annex) (January 2015)

30

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009040913 A

- JP 2022131236 A